(19) Europäisches Patentamt
European Patent Office
Office européen des brevets

(11) EP 0 513 700 B1

(12) **EUROPÄISCHE PATENTSCHRIFT**

(45) Veröffentlichungstag und Bekanntmachung des Hinweises auf die Patenterteilung:
**23.07.1997 Patentblatt 1997/30**

(51) Int. Cl.$^6$: **C07D 513/04**, A61K 31/425
// (C07D513/04, 277:00, 235:00)

(21) Anmeldenummer: **92107841.6**

(22) Anmeldetag: **09.05.1992**

(54) **Verfahren zur Herstellung von Imidazothiazolon-Derivaten**

Process for the preparation of imidazothiazolone derivates

Procédé de préparation de dérivés de imidazothiazolone

(84) Benannte Vertragsstaaten:
**CH DE FR GB IT LI**

(30) Priorität: **17.05.1991 DE 4116157**

(43) Veröffentlichungstag der Anmeldung:
**19.11.1992 Patentblatt 1992/47**

(73) Patentinhaber: **MERCK PATENT GmbH**
**D-64271 Darmstadt (DE)**

(72) Erfinder:
- **Schwarz, Michael, Dr.**
  **W-6080 Gross-Gerau 3 (DE)**
- **Casutt, Michael, Dr.**
  **W-6148 Heppenheim a.d.B (DE)**

(56) Entgegenhaltungen:
**EP-A- 0 242 686** **WO-A-85/02187**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 513 700 B1

Printed by Rank Xerox (UK) Business Services
2.14.11/3.4

**Beschreibung**

Die Erfindung betrifft ein neues Verfahren zur Herstellung eines Imidazothiazolon-Derivates der Formel I

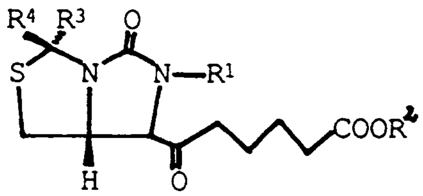

I,

worin

| | |
|---|---|
| $R^1$ | H oder eine für Stickstoff geeignete Schutzgruppe, |
| $R^2$ | H oder Alkyl mit 1-5 C-Atomen und |
| $R^3$ und $R^4$ | jeweils unabhängig voneinander H, Alkyl mit 1-5 C-Atomen, Cycloalkyl mit 3-7 C-Atomen, Aryl mit 6-10 C-Atomen, Heteroaryl mit 5-9 C-Atomen oder Aralkyl mit 7-10 C-Atomen oder zusammengenommen Alkylen oder Heteroalkylen mit jeweils 3-7 C-Atomen, |

bedeuten.

Verfahren zur Herstellung dieser Verbindungen sind z.B. aus DE 36 13 245 A1 und DE 37 03 872 A1 bekannt. Sie können als Zwischenprodukte zur Herstellung von D-(+)-Biotin verwendet werden.

Bei diesen bekannten Verfahren wird z.B. ein Phosphor-Ylid der Formel II

$$Ph_3P^+\text{-}\bar{C}H\text{-}(CH_2)_3\text{-}COOR^2 \qquad \text{II,}$$

worin $R^2$ die angegebene Bedeutung hat und Ph Phenyl bedeutet, mit einem Imidazothiazolon-Derivat der Formel III

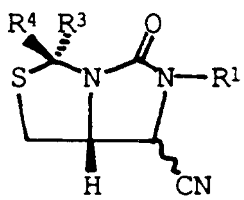

III,

worin $R^1$, $R^3$ und $R^4$ die angegebenen Bedeutungen haben, umgesetzt.

Das Ylid II wird aus einem Phosphoniumhalogenid der Formel IV

$$X^{\ominus}\ Ph_3P^+\text{-}CH_2\text{-}(CH_2)_3\text{-}COOR^2 \qquad \text{IV,}$$

worin X Br oder Cl bedeutet und Ph sowie $R^2$ die angegebenen Bedeutungen haben, hergestellt. Die Reaktionsbedingungen, insbesondere die zur Erzeugung des Ylids, gestatten jedoch nur Umsetzungen mit mäßigen Ausbeuten.

Der Erfindung lag die Aufgabe zugrunde, ein verbessertes Verfahren zur Herstellung der Imidazothiazolon-Derivate der Formel I zu entwickeln.

Es wurde nun überraschenderweise gefunden, daß bei der Überführung von IV in II durch Einsatz von Lithiumhexamethyldisilazid sowie durch Änderung der Reaktionsbedingungen die Ausbeute und damit die Effektivität dieses Reaktionsschrittes erheblich verbessert werden kann.

Die Herstellung von Lithiumhexamethyldisilazid unter anderen Versuchsbedingungen ist in WO 85/ 02187 beschrieben.

Gegenstand der Erfindung ist daher ein Verfahren zur Herstellung eines Imidazothiazolon-Derivats der Formel I durch Umsetzung eines Phosphor-Ylids der Formel II mit einem Imidazothizolon-Derivt der Formel III,
dadurch gekennzeichnet, daß man die Verbindung der Formel II ausgehend von einem Phosphoniumhalogenid der Formel IV durch Umsetzung mit Lithiumhexamethyldisilazid herstellt.

In den Formeln I und II bedeutet der Rest $R^1$ H oder eine für Stickstoff geeignete Schutzgruppe. Der Ausdruck "für Stickstoff geeignete Schutzgruppe" ist allgemein bekannt und bezieht sich auf Gruppen, die geeignet sind, das Stickstoffatom im Molekül der Verbindung I vor chemischen Umsetzungen zu schützen (zu blockieren), die aber leicht entfernbar sind, nachdem die gewünschte chemische Reaktion an einer anderen Stelle des Moleküls durchgeführt worden ist. Da die Schutzgruppen nach der gewünschten Reaktion oder Reaktionsfolge entfernt werden, ist ihre Art und Größe im übrigen nicht kritisch. Eine bevorzugte Schutzgruppe $R^1$ ist unsubstituiertes Benzyl. $R^1$ kann z.B. aber auch bedeuten: durch eine oder zwei $C_1$-$C_4$-Alkyl- und/oder $C_1$-$C_4$-Alkoxygruppen substituiertes Benzyl, daneben auch $C_3$-$C_5$ Alk-2-enyl oder $C_3$-$C_6$ Trialkylsilyl. Bei mehrfacher, vorzugsweise zweifacher Substitution eines Phenylrings sind die Substituenten vorzugsweise gleich, sie können aber auch verschieden sein. Sie befinden sich vorzugsweise in 4- und/oder 2-Stellung, sie können aber auch in 3-, 5- und/oder 6-Stellung stehen.

Vorzugsweise bedeutet der Rest $R^2$ Wasserstoff, Methyl oder Ethyl. Die Reste $R^3$ und $R^4$ können zusammengenommen Alkylen oder Heteroalkylen mit 3-7 C-Atomen sein, sind aber vorzugsweise jeweils unabhängig voneinander H, Alkyl, insbesondere Methyl oder Ethyl, Aryl, insbesondere Phenyl oder Aralkyl, insbesondere Benzyl. Besonders bevorzugt ist einer dieser Reste H, der andere Phenyl.

X ist vorzugsweise Br.

Verbindungen der Formel IV können nach an sich bekannten Methoden, wie sie z.B. aus Houben-Weyl, Methoden der Organischen Chemie Bd. 5/16, Bd. 12/1 oder Bd. 12/2 (Thieme Verlag, Stuttgart 4. Aufl.) bekannt sind, hergestellt werden.

Die Herstellung von II wird mittels Lithiumhexamethyldisilazid vorgenommen. Das Lithium-Reagenz wird hergestellt, indem man Hexamethyldisilazan mit einem Äquivalent n-Butyllithium in n-Hexan bei Temperaturen zwischen -10 bis 25°, bevorzugt bei 10-15° über eine Zeitdauer von 1-60 Minuten, bevorzugt 10-20 Minuten in einer Schutzgasatmosphäre behandelt; es wird in der Regel in situ verwendet.

So kann man anschließend die Reaktionsmischung unverdünnt oder durch ein inertes Lösungsmittel, vorzugsweise Tetrahydrofuran (THF), Hexan oder Pentan, verdünnt, zu einer Lösung oder Suspension von IV zutropfen. Als Lösungs- oder Suspensionsmittel von IV eignen sich beispielsweise die genannten.

Die Reaktionszeit beträgt 0,5-3 Stunden, während Temperaturen zwischen 0° und 30° empfehlenswert sind.

Die Nitrile der Formel III können nach an sich bekannten Methoden, wie sie beispielsweise in DE 36 13 245 A1 und DE 37 03 872 A1 sowie den darin erwähnten Literaturstellen beschrieben sind, hergestellt werden.

Zur Umsetzung von II mit III wird zweckmäßig ein Reaktionspartner vorgelegt, während man die zweite Komponente tropfenweise bei einer Temperatur zwischen 0° und 50°, vorzugsweise bei 20°-30°, zugibt und anschließend zur Vervollständigung der Reaktion rührt, vorzugsweise etwa 5-180 Minuten bei Raumtemperatur.

Das Nitril III wird hierzu in einem inerten Lösungsmittel, z.B. einem Ether, vorzugsweise Diethylether, THF, Dioxan, einem Keton, wie Aceton, Diethylketon oder Methylisobutylketon oder einem Kohlenwasserstoff, wie Hexan, Pentan oder Cyclohexan oder in einem Gemisch der genannten Lösungsmittel gelöst.

Besonders bevorzugt löst man III in THF oder einer Mischung aus THF und Hexan und versetzt tropfenweise bei einer Temperatur zwischen 20° und 30° mit einer verdünnten Lösung von II in einer Mischung aus THF und Hexan.

Die so erhaltenen Verbindungen der Formel I können durch an sich bekannte Veresterung oder Verseifung ineinander umgewandelt werden und gemäß der Angaben in DE-36 13 245 A1 und DE-37 03 872 A1 zu D-(+)-Biotin weiter umgesetzt werden.

Das erfindungsgemäße Verfahren erlaubt somit eine verbesserte Herstellung des D-(+)-Biotin-Vorproduktes I unter Verwendung von leicht zugänglichen wohlfeilen Ausgangsstoffen in hohen Ausbeuten.

In den nachfolgenden Beispielen bedeutet "übliche Aufarbeitung":

Man gibt, falls erforderlich, Wasser oder verdünnte Mineralsäure hinzu, extrahiert mit einem organischen Lösungsmittel wie Ethylacetat, Chloroform oder Dichlormethan, trennt ab, trocknet die organische Phase über Natriumsulfat, filtriert, behandelt die organische Phase mit Kieselgel/Aktivkohle, filtriert erneut, dampft ein und reinigt durch Chromatographie und/oder Kristallisation.

Vor- und nachstehend sind alle Temperaturen in Celsiusgraden angegeben.

## Beispiel 1

Zu 275 ml unter einer $N_2$-Atmosphäre vorgelegten Lösung von n-Butyllithium in n-Hexan (1,6 molar) werden bei 15° 74,3 g Hexamethyldisilazan zugetropft und 15 Minuten unter Erwärmung auf Raumtemperatur gerührt. Nach Verdünnung mit 200 ml THF wird die Reaktionsmischung, die Lithiumhexamethylsilazid enthält, tropfenweise zu einer Suspension von 88,7 g 4-Carboxybutyl-triphenylphosphoniumbromid in 300 ml THF gegeben und weitere 15 Minuten bei Raumtemperatur gerührt. Die Lösung enthält jetzt das entsprechende Ylid.

Anschließend werden wiederum bei Raumtemperatur zu dieser Lösung 50,3 g einer Mischung aus (7R,7aR)- und (7S,7aR)-3-Phenyl-6-benzyl-7-cyan-7,7a-dihydro-1H,4H-imidazo[1,5-c]thiazol-5(6H)-on, gelöst in 150 ml THF getropft. Man rührt noch 1,5 Stunden und erhält nach üblicher Aufarbeitung 46,0 g 3-Phenyl-6-benzyl-7-(5-carboxy-1-oxo-pentyl)-7,7a-dihydro-1H,3H-imidazo[1,5-c]-thiazol-5(6H)-on.

**Beispiel 2**

Analog Beispiel 1 erhält man ausgehend von 92,4 g 4-Methoxycarbonylbutyl-triphenylphosphoniumbromid durch Reaktion mit Menge Lithiumhexamethylsilazid und anschließende Umsetzung des Reaktionsproduktes mit 51,2 g einer Mischung aus (7R,7aR)- und (7S,7aR)-3-Phenyl-6-benzyl-7-cyan-7,7a-dihyro-1H,3H-imidazo[1,5-c]thiazol-5(6H)-on 47,3 g 3-Phenyl-6-benzyl-7-(5-methoxycarbonyl-1-oxo-pentyl)-7,7a-dihydro-1H,3H-imidazo[1,5-c]thiazol-5(6H)-9-on.
$[\alpha]_D^{25}$ = -171,3°, c = 1 (Benzol).

**Beispiel 3**

Analog Beispiel 1 erhält man ausgehend von 99,7 g 4-Ethoxycarbonylbutyl-triphenylphosphoniumbromid durch Reaktion mit Lithiumhexamethylsilazid und anschließende Umsetzung des Reaktionsproduktes mit 53,7 g einer Mischung aus (7R,7aR)- und (7S,7aR)-3-Phenyl-6-benzyl-7-cyan-7,7a-dihydro-1H,3H-imidazo[1,5-c]thiazol-5(6H)-on 48,7 g 3-Phenyl-6-benzyl-7-(5-ethoxycarbonyl-1-oxo-pentyl)-7,7a-dihydro-1H,3H-imidazo[1,5-c]thiazol-5(6H)-9-on.
$[\alpha]_D^{25}$ = -170,0°, c = 1 (Benzol).

**Patentansprüche**

1. Verfahren zur Herstellung eines Imidazothiazolon-Derivates der Formel I

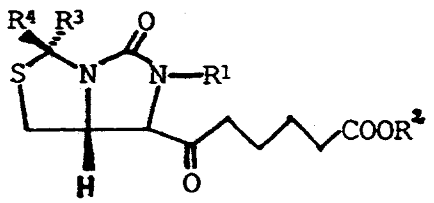

I,

worin

| | |
|---|---|
| $R^1$ | H oder eine für Stickstoff geeignete Schutzgruppe, |
| $R^2$ | H oder Alkyl mit 1-5 C-Atomen und |
| $R^3$ und $R^4$ | jeweils unabhängig voneinander H, Alkyl mit 1-5 C-Atomen, Cycloalkyl mit 3-7 C-Atomen, Aryl mit 6-10 C-Atomen, Heteroaryl mit 5-9 C-Atomen oder Aralkyl mit 7-10 C-Atomen oder zusammengenommen Alkylen oder Heteroalkylen mit jeweils 3-7 C-Atomen |

bedeuten,
durch Umsetzung eines Phosphor-Ylids der Formel II

$$Ph_3P^+\text{-}\bar{C}H\text{-}(CH_2)_3\text{-}COOR^2 \qquad II,$$

worin $R^2$ die angegebene Bedeutung hat und Ph Phenyl bedeutet,
mit einem Imidazothiazolon-Derivat der Formel III

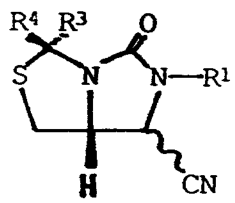

III,

worin $R^1$, $R^3$ und $R^4$ die angegebenen Bedeutungen haben,
dadurch gekennzeichnet, daß man die Verbindung der Formel II ausgehend von dem Phosphoniumhalogenid der Formel IV

$$X^- Ph_3^+\text{-}CH_2\text{-}(CH_2)_3\text{-}COOR^2 \qquad \text{IV,}$$

durch Umsetzung mit Lithiumhexamethyldisilazid, das durch Reaktion von Hexamethyldisilazan mit n-Butyllithium in n-Hexan unter Schutzgasatmosphäre bei Temperaturen zwischen -10 und 25° erhalten wird, herstellt.

## Claims

**1.** Process for the preparation of an imidazothiazolone derivative of formula I:

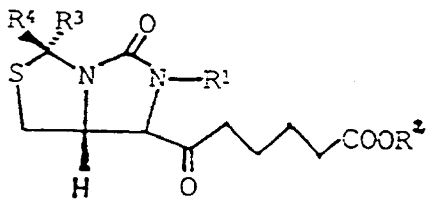

I

wherein

$R^1$ is H or a protecting group suitable for nitrogen,
$R^2$ is H or alkyl having 1-5 C atoms and
$R^3$ and $R^4$ in each case independently of one another are H, alkyl having 1-5 C atoms, cycloalkyl having 3-7 C atoms, aryl having 6-10 C atoms, heteroaryl having 5-9 C atoms or aralkyl having 7-10 C atoms, or taken together are alkylene or heteroalkylene having 3-7 C atoms in each case,

by reacting a phosphorus ylide of formula II:

$$Ph_3P^+\text{-}\bar{C}H\text{-}(CH_2)_3\text{-}COOR^2 \qquad \text{II}$$

wherein $R^2$ is as defined and Ph is phenyl, with an imidazothiazolone derivative of formula III:

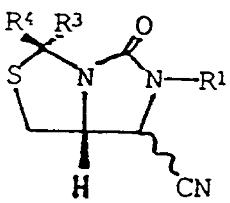

III

wherein $R^1$, $R^3$ and $R^4$ are as defined, characterised in that the compound of formula II is prepared from the phosphonium halide of formula IV:

$$X^- Ph_3^+\text{-}CH_2\text{-}(CH_2)_3\text{-}COOR^2 \qquad \text{IV}$$

by reaction with lithium hexamethyldisilazide which is obtained by reaction of hexamethyldisilazane with n-butyllithium in n-hexane under a protective gas atmosphere at temperatures between -10 and 25°.

## Revendications

**1.** Procédé de préparation d'un dérivé d'imidazothiazolone de formule I

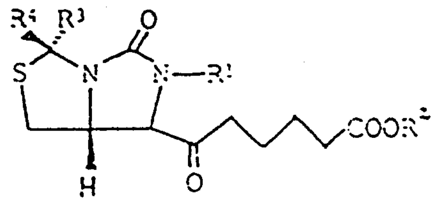

I,

dans laquelle

| | |
|---|---|
| $R^1$ | représente H ou un groupe protecteur approprié pour l'azote, |
| $R^2$ | représente H ou un alkyle en $C_1$ à $C_5$ et |
| $R^3$ et $R^4$ | représentent chacun indépendamment l'un de l'autre H, un alkyle en $C_1$ à $C_5$ , un cycloalkyle en $C_3$ à $C_7$ , un aryle en $C_6$ à $C_{10}$ , un hétéroaryle en $C_5$ à $C_9$ ou un aralkyle en $C_7$ à $C_{10}$ ou, pris ensemble, représentent un alkylène ayant à chaque fois de 3 à 7 atomes de carbone, |

par réaction d'un phosphor-ylide de formule II

$$Ph_3P^+ - {}^-CH-(CH_2)_3-COOR^2 \qquad II,$$

dans laquelle $R^2$ a la signification indiquée et Ph représente un phényle, avec un dérivé d'imidazothiazolone de formule III

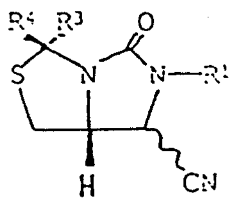

III,

dans laquelle $R^1$, $R^3$ et $R^4$ ont les significations indiquées, caractérisé en ce qu'on prépare le composé de formule II en partant de l'halogénure de phosphonium de formule IV

$$X^- \; Ph_3{}^+-CH_2-(CH_2)_3-COOR^2 \qquad IV,$$

par réaction avec de l'hexaméthyldisilazide de lithium, que l'on obtient par réaction d'hexaméthyldisilazane avec du n-butyllithium dans le n-hexane sous une atmosphère de gaz protecteur à des températures comprises entre -10 et 25°.